# EUROPEAN PATENT APPLICATION

(11) **EP 3 354 344 A1**
(43) Date of publication of application: **01.08.2018**
(21) Application number: 16848429.3
(22) Date of filing: 18.08.2016
(51) Int. Cl.: B01L 3/02, G01N 1/00, G01N 35/10

(54) **ATTACHMENT STRUCTURE FOR PIPETTE OR PIPETTE TIP AND ATTACHMENT FOR LIQUID INJECTION, AND ATTACHMENT FOR LIQUID INJECTION**

(30) Priority: 25.09.2015 JP 2015188466
(71) Applicant: NOK Corporation, Minato-ku Tokyo 105-8585 (JP)
(72) Inventor: FUTASHIMA Ryo, Fujisawa-shi Kanagawa 251-0042 (JP); MUROTA Yuki, Fujisawa-shi Kanagawa 251-0042 (JP); UDA Toru, Fujisawa-shi Kanagawa 251-0042 (JP)
(74) Representative: Nordic Patent Service A/S
(86) International application number: PCT/JP2016/074161
(87) International publication number: WO 2017/051641

(57) **Abstract**

The present invention addresses the problem of providing an attachment structure that ensures a large crushing amount of a fluid injecting attachment for sealing around an inlet, in which the fluid injecting attachment will not be deformed or damaged. This problem is solved by an attachment structure of a pipette 100 and a fluid injecting attachment 1, which includes a first cylindrical hole 21 into which a tip end portion 101 having a tapered shape of the pipette 100 is inserted, on one end side of an attachment main body 2 formed in a cylindrical shape by a rubber elastic body, and a second cylindrical hole 22 that is communicated with the first cylindrical hole 21 and out through which fluid inside the pipette 100 flows, on the other end side, and a step portion 23 between the first cylindrical hole 21 and the second cylindrical hole 22 due to the second cylindrical hole 22 being formed with a smaller diameter than the first cylindrical hole 21, in which a washer 3 is provided on the step portion 23, the tip end portion 101 of the pipette 100 is inserted through the first cylindrical hole 21 and inserted into the washer 3, and while the tip end portion 101 is in a state inserted into the washer 3, the second cylindrical hole 22 has a crush allowance 24 farther toward a tip end side than the tip end portion 101.

## Description

### TECHNICAL FIELD

The present invention relates to an attachment structure of a pipette or a pipette tip and a fluid injecting attachment, and a fluid injecting attachment. More particularly, the present invention relates to an attachment structure of a pipette or a pipette tip and a fluid injecting attachment, and a fluid injecting attachment, in which a large crushing amount of a fluid injecting attachment for sealing around an inlet is able to be ensured, and the fluid injecting attachment will not be deformed or damaged.

### BACKGROUND

Known by names such as micro total analysis systems (µTAS) or Lab-on-a-chip, microfluidic chips that are provided with microscopic structures such as ports and microchannels that form flow paths of predetermined shapes inside a substrate, and which perform various operations such as chemically reacting, synthesizing, purifying, extracting, producing and/or breaking down substances within the microscopic structures, are receiving attention. Microfluidic chips are expected to be applied to a wide range of uses such as medical related markets such as genomic analysis, genomic drug discovery, protein analysis, preventive diagnosis, clinical diagnosis or drug screening, and chemical analysis, food analysis or environmental monitoring.

In the past, various methods have been proposed that enable a fluid such as a reagent to be injected from a pipette into a microchannel in a microfluidic chip through an inlet, and enable a seal to be created by a simple structure, without using a solution sending tube, a connector, a pump, and a valve and the like.

In Figure 7(d) of Patent Document 1, an abutting portion of a flexible member is provided at a tip end of a pipette, and a seal is created by abutting this abutting portion against a pipette receiving portion formed by only a rigid member.

Also, attaching an attachment formed entirely by a flexible elastomer to a tip end portion of a dispensing tip to seal around an inlet when injecting fluid has also been proposed, in Figure 3 of Patent Document 2. The attachment has a hole formed in the center portion and is able to be attached to the tip end portion of the dispensing tip using the elasticity of the elastomer, by inserting the tip end portion of the dispensing tip into this hole.

Furthermore, fixing a port that has a cylindrical shape and is made of elastomeric materials to an open inlet port of a microfluidic test device has also been proposed, in Figure 1A to Figure 1E of Patent Document 3. The port has a first portion, a second portion, a third portion, and a base portion, all having different inside diameters, so even if hollow tubes having various outside diameters are used, the port will closely contact the outer peripheral surfaces of the hollow tubes so a seal is able to be created.

### PRIOR ART DOCUMENTS

### PATENT DOCUMENTS

Patent Document 1: JP-A-2006-322850
Patent Document 2: JP-A-2012-147751
Patent Document 3: National Publication of International Patent Application No. 2015-518571

### SUMMARY OF THE INVENTION

### PROBLEM TO BE SOLVED BY THE INVENTION

When injecting fluid from the pipette into the inlet, it is necessary to increase the crushing amount of the flexible member (the elastomer) provided on the pipette as described in Patent Documents 1 and 2, so as to stably seal around the inlet. Also, when using an automatic injection device that has a plurality of pipettes, variation (tolerance) in the distance between the tip end portions of the pipettes and the inlets may increase, so it is necessary to increase the crushing amount of the flexible member (the elastomer) to absorb this variation as well.

However, when an abutting portion formed by a flexible member is provided on the tip end of the pipette as described in Patent Document 1, the volume of the flexible member itself is small so it is difficult to ensure a large crushing amount, and consequently, variation may not be able to be sufficiently absorbed which is problematic.

On the other hand, when an attachment that is made entirely of an elastomer is attached to the tip end portion of the pipette as described in Patent Document 2, a sufficient crushing amount is able to be ensured, but when the attachment is pushed toward the inlet to obtain the predetermined crushing amount, the hole in the attachment may deform and the holding force may be lost, or the attachment itself may be damaged, due to the tip end portion of the pipette being excessively pressed into the hole of the attachment.

Therefore, the present invention addresses the problem of providing an attachment structure of a pipette or a pipette tip and a fluid injecting attachment, and a fluid injecting attachment, in which a large crushing amount of a fluid injecting attachment for sealing around an inlet is able to be ensured, and the fluid injecting attachment will not be deformed or damaged.

Other problems of the present invention will be apparent from the following description.

### MEANS FOR SOLVING PROBLEM

The above-described problems are solved by the following respective inventions.

1. An attachment structure of a pipette or a pipette tip attached to the pipette, and a fluid injecting attachment that is detachably attached to the pipette or the pipette tip, wherein:
   the fluid injecting attachment has a first cylindrical hole into which a tip end portion having a tapered shape of the pipette or the pipette tip is inserted, on one end side of an attachment main body formed in a cylindrical shape by a rubber elastic body, and a second cylindrical hole that is communicated with the first cylindrical hole and out through which fluid inside the pipette or the pipette tip flows, on the other end side, and a step portion between the first cylindrical hole and the second cylindrical hole due to the second cylindrical hole being formed with a smaller diameter than the first cylindrical hole;
   a washer is provided on the step portion;
   the tip end portion of the pipette or the pipette tip is inserted through the first cylindrical hole and inserted into the washer; and
   while the tip end portion is in a state inserted into the washer, the second cylindrical hole has a crush allowance farther toward a tip end side than the tip end portion.
2. The attachment structure of the pipette or the pipette tip and the fluid injecting attachment according to 1, wherein the washer is made of synthetic resin.
3. An attachment structure of a pipette or a pipette tip attached to the pipette, and a fluid injecting attachment that is detachably attached to the pipette or the pipette tip, wherein:
   the fluid injecting attachment has a first cylindrical hole into which a tip end portion having a tapered shape of the pipette or the pipette tip is inserted, on one end side of an attachment main body formed in a cylindrical shape by a rubber elastic body, and a second cylindrical hole that is communicated with the first cylindrical hole and out through which fluid inside the pipette or the pipette tip flows, on the other end side, and a flange portion having a smaller diameter than an inside diameter of the first cylindrical hole and a larger diameter than an inside diameter of the second cylindrical hole, on the tip end portion of the pipette or the pipette tip;
   the tip end portion of the pipette or the pipette tip is inserted through the first cylindrical hole and the flange portion is abutted against the step portion; and
   while the flange portion is in a state abutted against the step portion, the second cylindrical hole has a crush allowance farther toward a tip end side than the tip end portion.
4. A fluid injecting attachment that is detachably attached to a pipette or a pipette tip attached to the pipette, comprising:
   a first cylindrical hole into which a tip end portion having a tapered shape of the pipette or the pipette tip is inserted, on one end side of an attachment main body formed in a cylindrical shape by a rubber elastic body, and a second cylindrical hole that is communicated with the first cylindrical hole and out through which fluid inside the pipette or the pipette tip flows, on the other end side; and
   a step portion between the first cylindrical hole and the second cylindrical hole due to the second cylindrical hole being formed with a smaller diameter than the first cylindrical hole,
   wherein a washer into which the tip end portion of the pipette or the pipette tip is inserted is provided on the step portion.
5. The fluid injecting attachment according to 4, wherein an outside diameter of the washer is slightly larger than an inside diameter of the first cylindrical hole.

### EFFECT OF THE INVENTION

The present invention is able to provide an attachment structure of a pipette or a pipette tip and a fluid injecting attachment, and a fluid injecting attachment, in which a large crushing amount of a fluid injecting attachment for sealing around an inlet is able to be ensured, and the fluid injecting attachment will not be deformed or damaged.

### BRIEF DESCRIPTION OF DRAWINGS

[Figure 1] Figure 1 is a perspective view of one example of a fluid injecting attachment according to the present invention being used on a pipette.
[Figure 2] Figure 2 is a view of an example of each of a micropipette and a pipette tip.
[Figure 3] Figure 3 is a sectional explanatory view of an attachment structure of the fluid injecting attachment shown in Figure 1.
[Figure 4] Figure 4 is a sectional view of a usage state of the attachment structure of the fluid injecting attachment shown in Figure 1.
[Figure 5] Figure 5 is a sectional view of a usage state of another example of the fluid injecting attachment according to the present invention.
[Figure 6] Figure 6 is a sectional view of a usage state of another example of the fluid injecting attachment according to the present invention.
[Figure 7] Figure 7 is a sectional view of a usage state of yet another example of the fluid injecting attachment according to the present invention.

### DESCRIPTION OF THE PREFERRED EMBODIMENTS

Hereinafter, the preferred embodiments of the present invention will be described in detail with reference to the drawings.

The attachment structure of the fluid injecting attachment according to the present invention is used when performing a fluid injecting operation using a pipette, with respect to an inlet of a microchannel or the like of a microfluidic chip into which fluid is to be introduced.

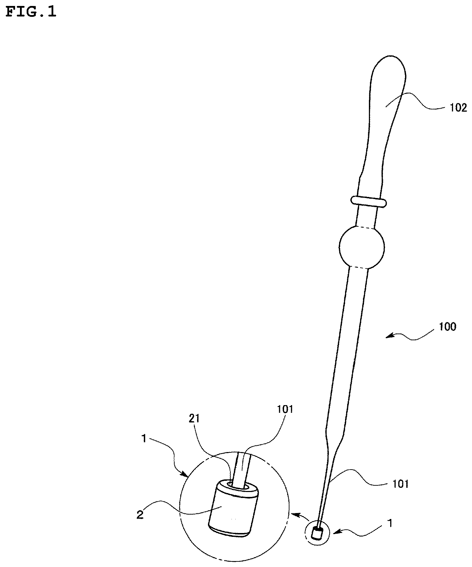

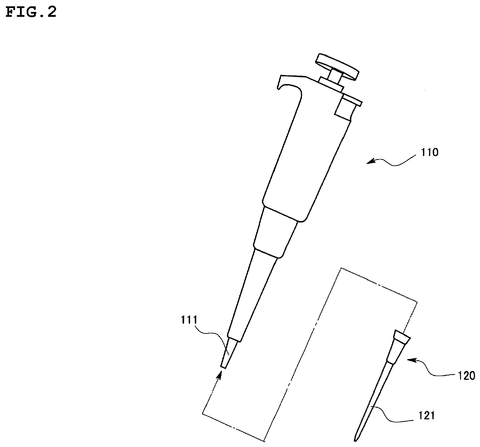

Aside from a plurality of pipettes provided on an automatic injection device, not shown, the pipette may be, for example, a pipette 100 that sucks and injects fluid by a rubber bulb 102, as shown in Figure 1, or a micropipette 110 that is used when sucking and injecting fluid of extremely small amounts in microliter units, as shown in Figure 2. The pipette of the present invention includes both of these.

A fluid injecting attachment 1 (hereinafter, simply referred to as attachment) is used detachably attached to a tip end portion 101 of the pipette 100 or a tip end portion 111 of the micropipette 110 but is not limited to these uses. For example, as shown in Figure 2, the attachment 1 may also be used detachably attached to a tip end portion 121 of a pipette tip 120 that is attached to a tip end of the pipette 100 or the micropipette 110. The tip end portions 101 and 111 of the pipettes 100 and 110 and the tip end portion 121 of the pipette tip 120 are formed in tapered shapes by injection molding or the like with thermoplastic resin as the molding material. Tip end portions that are commercially available may be used.

Hereinafter, in this specification, a case in which the attachment 1 is detachably attached to the tip end portion 101 of the pipette 100 that sucks and injects fluid using the rubber bulb 102, as shown in Figure 1, will be described. However, the description below may also incorporate a case in which the attachment 1 is detachably attached to the tip end portion 111 of the micropipette 110 and the tip end portion 121 of the pipette tip 120 shown in Figure 2.

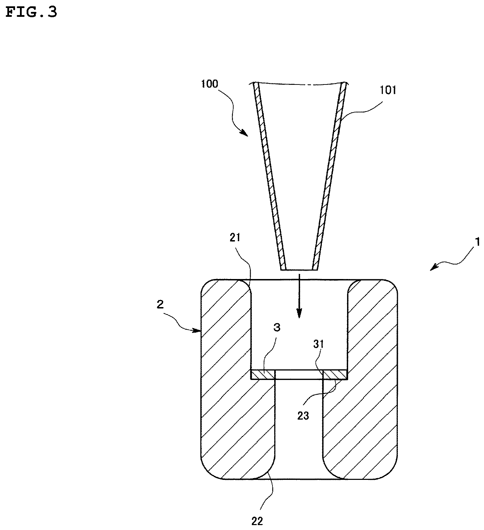

Figure 1 is a perspective view of the fluid injecting attachment according to the present invention in a state attached to the tip end portion of the pipette, and shows the portion encircled by the alternate long and short dash line enlarged. Figure 3 is an enlarged sectional view of the fluid injecting attachment shown in Figure 1.

The entire attachment 1 is formed in a cylindrical shape by a rubber elastic body. The specific rubber elastic body differs depending on the type of fluid used and the like, but material of rubber or elastomer is typically selected. Representative examples include silicone rubber, fluororubber, acrylic rubber, nitrile rubber, and butyl rubber and the like. Compression molding or injection molding, for example, may be employed as the method for forming the cylindrical shape.

The attachment 1 includes, for example, an attachment main body 2 formed in a circular cylindrical shape as shown in Figure 3, and a washer 3 provided inside the attachment main body 2.

The attachment main body 2 has a first cylindrical hole 21 into which the tip end portion 101 of the pipette 100 is inserted, on one end side (the upper side in Figure 3), and a second cylindrical hole 22 out through which fluid in the pipette 100 flows, on the other end side (the lower side in Figure 3). The first cylindrical hole 21 and the second cylindrical hole 22 are communicated in the axial direction of the attachment main body 2, forming a through-hole from one end side to the other end side of the attachment main body 2.

The first cylindrical hole 21 is a hole having a circular cylindrical shape with a constant inside diameter in the axial direction (the vertical direction in Figure 3) of the attachment main body 2, and has an inside diameter sufficient for the tip end portion 101 of the pipette 100 to be able to be inserted.

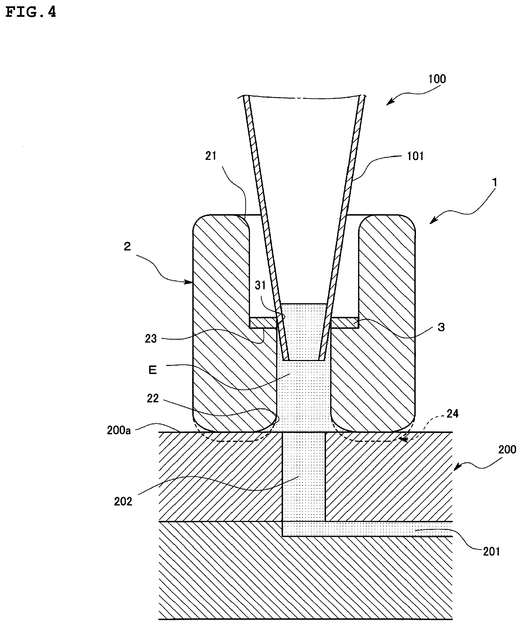

More specifically, the first cylindrical hole 21 is formed such that the outer peripheral surface of the tip end portion 101 will essentially not pressure contact the inside surface of the first cylindrical hole 21 in the radial direction while the tip end portion 101 is in an attached state in which it is inserted into the washer 3, described later, and is unable to be inserted any further, as shown in Figure 4. That is, the first cylindrical hole 21 is formed such that, in the attached state, the inside surface of the first cylindrical hole 21 just contacts the outer peripheral surface of the tip end portion 101, or does not contact the outer peripheral surface of the tip end portion 101, as shown in Figure 4.

On the other hand, the second cylindrical hole 22 is also a hole having a circular cylindrical shape with a constant inside diameter in the axial direction of the attachment main body 2, but has a smaller diameter than the first cylindrical hole 21. Therefore, there is a step at the boundary portion of the first cylindrical hole 21 and the second cylindrical hole 22. In the present invention, a step portion 23 is formed by a surface that is parallel to the surface on the other surface side (by a surface that is orthogonal to the axial direction of the cylindrical hole 22).

The axial length of the second cylindrical hole 22 is formed having a crush allowance 24 farther toward the tip end side than the tip end portion 101, in the attached state shown in Figure 4 in which the tip end portion 101 of the pipette 100 is inserted into a mounting hole 31 in the washer 3.

The washer 3 is mounted to this step portion 23 by being inserted from the first cylindrical hole 21 side. The washer 3 shown in this embodiment is formed by a flat washer having a flat plate shape, and has the mounting hole 31 into which the tip end portion 101 of the pipette 100 is inserted formed in the center. Synthetic resin is preferably used as the material of the washer 3. This enables the attachment 1 to be lightweight and inexpensive. In particular, thermoplastic resin that is inexpensive and easy to form, such as polypropylene, may be used.

The outside diameter of the washer 3 is the same as, or slightly larger than, the inside diameter of the first cylindrical hole 21. When the outside diameter of the washer 3 is formed with a slightly larger diameter, the outside diameter of the washer 3 pressure contacts the inside surface of the first cylindrical hole 21, such that the washer 3 is elastically held on the step portion 23, which is preferable. This is preferable in view of the characteristics of a pipette and the like that uses a fluid such as a reagent, because the washer 3 is held elastically so there is no need to use means such as an adhesive.

Also, the mounting hole 31 in the washer 3 is large enough so that the tip end portion 101 of the pipette 100 is able to be inserted into it. More specifically, the mounting hole 31 may also be formed equal to or smaller than the inside diameter of the second cylindrical hole 22.

Figure 4 is a sectional view of the attachment structure of the pipette 100 and the attachment 1. The operation and effects of the attachment structure and the attachment 1 will now be described with reference to this drawing using as an example a case in which a fluid E is injected into an inlet 202 of a microchannel 201 formed in a microfluidic chip 200.

When injecting the fluid E that is in the pipette 100 into the microchannel 201 of the microfluidic chip 200, the attachment 1 is first attached to the tip end portion 101 of the pipette 100. That is, the tip end portion 101 is inserted through the first cylindrical hole 21 of the attachment 1, and then inserted into the mounting hole 31 in the washer 3. At this time, the attachment 1 is in an attached state in which it is held to the tip end portion 101 by the elasticity of the tip end portion 101 and/or the elasticity of the washer 3. The tip end of the tip end portion 101 passes through the washer 3 and reaches into the second cylindrical hole 22.

Then, the attachment 1 is pushed against an upper surface 200a of the microfluidic chip 200 such that the area around the second cylindrical hole 22 on the lower end of the attachment main body 2 abuts against the area around the inlet 202.

At this time, the attachment 1 is crushed by the load placed on the attachment 1. The load at this time is transmitted from the tip end portion 101 to the step portion 23 of the attachment main body 2 via the washer 3, and compresses and deforms the attachment main body 2, or more specifically, the rubber elastic body around the second cylindrical hole 22. As a result, reaction force from the compressive deformation in the axial direction is generated in the attachment main body 2, so the area around the inlet 202 is able to be reliably sealed.

Moreover, the inside surface of the first cylindrical hole 21 is essentially not in pressure contact in the radial direction with the outer peripheral surface of the tip end portion 101, so this load essentially does not act in the radial direction of the attachment main body 2. Therefore, when the load is applied to the attachment 1, the rubber elastic body around the second cylindrical hole 22 is able to be effectively compressed in the axial direction without force being applied in a direction that would tear the attachment 1. Also, the tip end portion 101 is inserted into the mounting hole 31 in the washer 3, so even if the load is applied, force that would deform the hole diameter will not act on the second cylindrical hole 22, and holding force against the tip end portion 101 will not be reduced.

Further, the reaction force of the attachment main body 2 counteracts the injection pressure when fluid is injected. Therefore, the attachment 1 maintains good sealing performance, and the fluid E can be introduced from the pipette 100 into the microchannel 201 through the second cylindrical hole 22 and the inlet 202 without the fluid E leaking out.

In this way, with the attachment structure and the attachment 1 according to the present invention, the reaction force generated by the compressive deformation in the axial direction of the attachment main body 2 in this way is effectively utilized when injecting fluid. The load transmitted from the tip end portion 101 to the washer 3 compresses and crushes the sufficiently large rubber elastic body that surrounds the second cylindrical hole 22 and protrudes farther toward the tip end side than the tip end portion 101, so a large crushing amount is able to be ensured. Therefore, even when the present invention is applied to an automatic injection device that uses a plurality of pipettes, variation (tolerance) with each pipette is able to be sufficiently absorbed.

Because the attachment 1 includes the attachment main body 2 that is formed entirely by the rubber elastic body, and the washer 3, the manufacturing process is able to be simplified and the manufacturing cost is able to be reduced. Also, the pipette 100 may be a commercially available pipette used as is, so the initial cost is also able to be reduced.

In this embodiment, the inside surface of the first cylindrical hole 21 is formed so as not to contact the tip end portion 101, but if the inside diameter of the first cylindrical hole 21 is formed so as to just contact the outer peripheral surface of the tip end portion 101, the tip end portion 101 is able to be held by the inside surface of the first cylindrical hole 21 and the washer 3, so the attachment 1 is able to be even more stably held to the tip end portion 101.

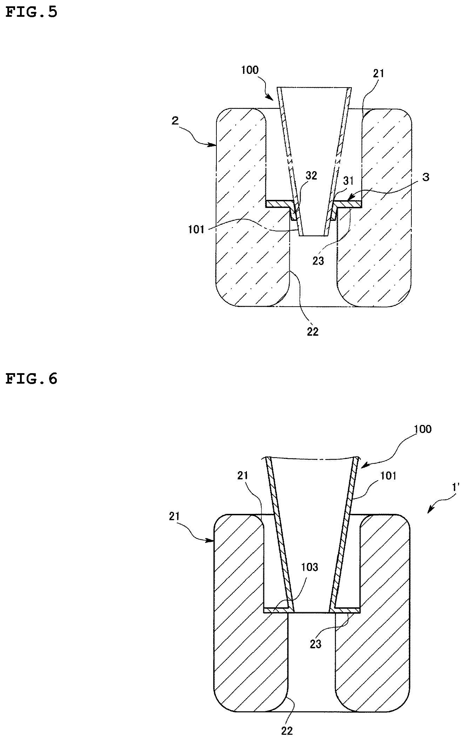

Also, in the embodiment described above, a flat washer is used as the washer 3, but the washer 3 may also be provided with a narrowing, funnel-shaped cylindrical portion 32 that gradually becomes narrower in diameter toward the tip end side (the downstream side in Figure 5), at the mounting hole 31, as shown in Figure 5. Accordingly, the contact area with the tip end portion 101 is ensured, which enables the holding state to be even better.

Moreover, when attaching this attachment 1 to the tip end portion 101 of the pipette 100, even if the central axis of the tip end portion 101 deviates from the central axis of the attachment main body 2, the tip end portion 101 is able to be smoothly guided by the funnel-shaped cylindrical portion 32. Therefore, an effect in which the work of attaching the attachment 1 to the tip end portion 101 is improved is able to be obtained, so the present invention is particularly effective when applied to an automatic injection device or an automatic fluid injection system that uses a pipetting robot.

Figure 6 is a sectional view of another example of the attachment structure of the fluid injecting attachment according to the present invention. Portions having the same reference numerals as those of the attachment structure and the fluid injecting attachment shown in Figures 1 to 4 are portions that have the same structure, so descriptions of these portions will be omitted here as they are cited in the description above.

With an attachment structure of this fluid injecting attachment 1' (hereinafter, referred to as attachment 1'), a washer is not provided on the step portion 23 of the attachment main body 2. Instead of the washer, a flange portion 103 that protrudes out in the radial direction is integrally provided on the tip end portion 101 of the pipette 100. This flange portion 103 abuts against the step portion 23 as a result of the tip end portion 101 of the pipette 100 being inserted through the first cylindrical hole 21.

The outside diameter of the flange portion 103 is the same as, or slightly larger than, the inside diameter of the first cylindrical hole 21. The tip end portion 101 is elastically held on the step portion 23 by pressure contact between this flange portion 103 and the inside surface of the first cylindrical hole 21.

With this attachment structure, when a load is applied, the flange portion 103 provided on the tip end portion 101 of the pipette 100 fulfills the same function as the washer 3 above, so this attachment structure displays the same effect as that described above in which the attachment main body 2 compresses in the axial direction via the step portion 23 without causing the attachment 1 to be deformed or damaged, and thus is able to create a seal by the reaction force at this time.

Also, the load transmitted from the tip end portion 101 to the step portion 23 via the flange portion 103 compresses and crushes the sufficiently large rubber elastic body that surrounds the second cylindrical hole 22 and protrudes farther toward the tip end side than the tip end portion 101, so a large crushing amount is able to be ensured just as described above. Therefore, even when the present invention is applied to an automatic injection device that uses a plurality of pipettes, variation (tolerance) with each pipette is able to be sufficiently absorbed.

With this attachment structure, even if a load is applied, it will not affect the holding state between the flange portion 103 and the inside surface of the first cylindrical hole 21, so the holding force with respect to the tip end portion 101 will not be reduced.

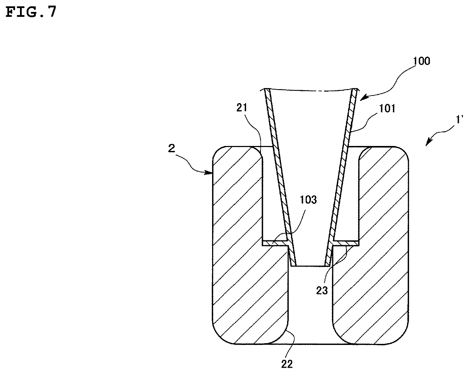

This flange portion 103 is provided on the tip end of the tip end portion 101, but the tip end of the tip end portion 101 may also protrude further than the flange portion 103, as shown in Figure 7. Accordingly, the tip end side of the tip end portion 101 reaches into the second cylindrical hole 22, so fluid is able to be injected even more smoothly.

In each of the embodiments described above, the attachment main body 2 is formed such that the outer shape is a circular cylindrical shape, but the outer shape does not necessarily have to be a circular cylindrical shape and may be an angular tube shape instead. Also, the outer peripheral surface of the attachment main body 2 may have a shape that is inclined in the axial direction.

Also, the first cylindrical hole 21 may also have a shape that gradually decreases in diameter toward the step portion 23. Furthermore, the second cylindrical hole 22 may also have a shape that gradually decreases or increases in diameter in the axial direction.

### EXPLANATIONS OF LETTERS OR NUMERALS

1, 1': FLUID INJECTING ATTACHMENT
2: ATTACHMENT MAIN BODY
   21: FIRST CYLINDRICAL HOLE
   22: SECOND CYLINDRICAL HOLE
   23: STEP PORTION
   24: CRUSH ALLOWANCE
3: WASHER
   31: MOUNTING HOLE
   32: FUNNEL-SHAPED CYLINDRICAL PORTION
100: PIPETTE
   101: TIP END PORTION
   102: RUBBER BULB
   103: FLANGE PORTION
110: MICROPIPETTE
120: PIPETTE TIP
   121: TIP END PORTION
200: MICROFLUIDIC CHIP
   200a: UPPER SURFACE
201: MICROCHANNEL
202: INLET
E: FLUID

## Claims

1. An attachment structure of a pipette or a pipette tip attached to the pipette, and a fluid injecting attachment that is detachably attached to the pipette or the pipette tip, wherein:
the fluid injecting attachment has a first cylindrical hole into which a tip end portion having a tapered shape of the pipette or the pipette tip is inserted, on one end side of an attachment main body formed in a cylindrical shape by a rubber elastic body, and a second cylindrical hole that is communicated with the first cylindrical hole and out through which fluid inside the pipette or the pipette tip flows, on the other end side, and a step portion between the first cylindrical hole and the second cylindrical hole due to the second cylindrical hole being formed with a smaller diameter than the first cylindrical hole;
a washer is provided on the step portion;
the tip end portion of the pipette or the pipette tip is inserted through the first cylindrical hole and inserted into the washer; and
while the tip end portion is in a state inserted into the washer, the second cylindrical hole has a crush allowance farther toward a tip end side than the tip end portion.

2. The attachment structure of the pipette or the pipette tip and the fluid injecting attachment according to claim 1, wherein the washer is made of synthetic resin.

3. An attachment structure of a pipette or a pipette tip attached to the pipette, and a fluid injecting attachment that is detachably attached to the pipette or the pipette tip, wherein:
the fluid injecting attachment has a first cylindrical hole into which a tip end portion having a tapered shape of the pipette or the pipette tip is inserted, on one end side of an attachment main body formed in a cylindrical shape by a rubber elastic body, and a second cylindrical hole that is communicated with the first cylindrical hole and out through which fluid inside the pipette or the pipette tip flows, on the other end side, and a flange portion having a smaller diameter than an inside diameter of the first cylindrical hole and a larger diameter than an inside diameter of the second cylindrical hole, on the tip end portion of the pipette or the pipette tip;
the tip end portion of the pipette or the pipette tip is inserted through the first cylindrical hole and the flange portion is abutted against the step portion; and
while the flange portion is in a state abutted against the step portion, the second cylindrical hole has a crush allowance farther toward a tip end side than the tip end portion.

4. A fluid injecting attachment that is detachably attached to a pipette or a pipette tip attached to the pipette, comprising:
a first cylindrical hole into which a tip end portion having a tapered shape of the pipette or the pipette tip is inserted, on one end side of an attachment main body formed in a cylindrical shape by a rubber elastic body, and a second cylindrical hole that is communicated with the first cylindrical hole and out through which fluid inside the pipette or the pipette tip flows, on the other end side; and
a step portion between the first cylindrical hole and the second cylindrical hole due to the second cylindrical hole being formed with a smaller diameter than the first cylindrical hole,
wherein a washer into which the tip end portion of the pipette or the pipette tip is inserted is provided on the step portion.

5. The fluid injecting attachment according to claim 4, wherein an outside diameter of the washer is slightly larger than an inside diameter of the first cylindrical hole.
